# EUROPÄISCHE PATENTSCHRIFT

(11) **EP 1 862 151 B1**
(45) Veröffentlichungstag und Bekanntmachung des Hinweises auf die Patenterteilung: **06.10.2010**
(21) Anmeldenummer: 06011218.2
(22) Anmeldetag: 31.05.2006
(51) Int. Cl.: A61F 2/46

(54) **Verfahren zur Auswahl eines Oberschenkelimplantats**
Method for selection of an femoral implant
Procédé de sélection d'un implant fémoral

(43) Veröffentlichungstag der Anmeldung: 05.12.2007
(73) Patentinhaber: BrainLAB AG, 85622 Feldkirchen (DE)
(72) Erfinder: Müller, Heiko, 80335 München (DE); Dick, Robert, 80686 München (DE); Adamski, Martin, 81667 München (DE); Tuma, Gregor, 81675 München (DE)
(74) Vertreter: Gassenhuber, Andreas

(56) Entgegenhaltungen:
- EP-A- 1 563 810
- DE-U1-202005 001 127
- DE-U1-202005 001 128
- US-A1- 2005 065 617

## Beschreibung

Die vorliegende Erfindung betrifft ein Verfahren und eine Vorrichtung zur Auswahl eines Oberschenkelimplantats, welches bei Hüftgelenksoperationen vorzugsweise auf einen Oberschenkelkopf aufzementiert wird, wobei mittels dem erfindungsgemäßen Verfahren und der erfindungsgemäßen Vorrichtung ein passendes Oberschenkelimplantat basierend auf virtuellen oder digitalen Modellen eines Oberschenkelhalses und/oder eines Oberschenkelkopfes eines Patienten ermittelt oder ausgewählt wird.

Bei Hüftgelenksoperationen wird üblicherweise der Oberschenkelhals beibehalten und der Oberschenkelkopf abgefräst, um einen Oberflächenersatz des Oberschenkelkopfes oder ein Oberschenkelimplantat auf oder in den Oberschenkelkopf auf- bzw. einzuzementieren. Wird ein zu kleines Oberschenkelimplantat oder ein Oberschenkelimplantat mit einem zu kleinen Innendurchmesser gewählt und aufzementiert, besteht die Gefahr, dass sich der Fräser zur Bearbeitung der lmplantatinnenkontur unbeabsichtigt in den Oberschenkelhals einfräst, so dass es zu einer Oberschenkelhalsfraktur kommen kann. Wird ein zu großes Oberschenkelimplantat gewählt, so schließt das Oberschenkelimplantat nicht ausreichend mit dem Oberschenkelkopf oder an dem Oberschenkelhals ab.

Bei herkömmlichen Verfahren wird die Implantatsgröße präoperativ mittels Röntgenstrahlen und Vorlagen bestimmt. Dieses Vorgehen ist zeitaufwändig und enthält eine Vielzahl möglicher Ungenauigkeiten aufgrund von Projektionsfehlern. Intraoperativ wird die bestimmte Größe mittels Messlehren oder Metallvorlagen überprüft, was einen zusätzlichen Zeitaufwand hervorruft.

Es ist eine Aufgabe der vorliegenden Erfindung die Nachteile aus dem Stand der Technik zu überwinden. Es ist weiter eine Aufgabe der vorliegenden Erfindung eine Vorrichtung und ein Verfahren bereitzustellen, welche eine schnelle und genaue Ermittlung eines passenden Oberschenkelimplantats ermöglichen.

Diese Aufgabe wird durch den Gegenstand der unabhängigen Ansprüche gelöst. Vorteilhafte Ausführungsformen ergeben sich aus den abhängigen Ansprüchen.

Bei dem erfindungsgemäßen Verfahren wird ein Oberschenkelimplantat oder ein Oberschenkelkopfersatz oder -aufsatz aus Modellen eines Oberschenkelhalses und/oder eines Oberschenkelkopfes eines Patienten ermittelt. Dabei werden berührungslos oder mittels Berührung oder Abtastung, zum Beispiel mittels eines Laser-Pointers oder eines mit mechanischen Berührungen arbeitenden Pointers, Referenzpunkte, insbesondere anatomische Landmarken, an oder auf dem Oberschenkelhals oder dem Oberschenkelkopf des Patienten erfasst. Dabei ist an dem Oberschenkelhals ein, insbesondere IR-Strahlung emittierender oder reflektierender Referenzstern angeordnet, welcher von einer Kamera, insbesondere einer IR-Kamera, eines Navigationssystems erfasst werden kann und dessen dreidimensionale Raumposition relativ zu einem Referenzkoordinatensystem mittels des Navigationssystems ermittelt werden kann. Aus den erfassten Referenzpunkten des Oberschenkelhalses und/oder des Oberschenkelkopfes kann das Navigationssystem basierend auf den ermittelten dreidimensionalen Raumpositionen des Referenzsterns dreidimensionale Referenzpunkt-Raumpositionen der erfassten Referenzpunkte relativ zu dem Referenzkoordinatensystem, wie dem Referenzstern oder dem Ursprung des Referenzsterns, ermitteln, so dass der Oberschenkelhals und/oder der Oberschenkelkopf bezüglich des Referenzkoordinatensystems, wie dem Referenzstern, registriert werden können.

Aus den ermittelten dreidimensionalen Referenzpunkt-Raumpositionen wird ein Modell des Oberschenkelhalses und/oder des Oberschenkelkopfes, wie ein virtuelles oder digitales Modell des Oberschenkelhalses und/oder des Oberschenkelkopfes, ermittelt. Zum Beispiel können die Modelle ermittelt werden, indem die ermittelten Referenzpunkt-Raumpositionen zu einer durchgehenden oder durchgängigen Form oder Oberfläche verbunden werden, oder indem aus den ermittelten Referenzpunkt-Raumpositionen eine Form oder Oberfläche des Oberschenkelhalses und/oder des Oberschenkelkopfes angenähert wird. Das Modell des proximalen Femur mit Oberschenkelkopf und Schenkelhals wird insbesondere dadurch ermittelt, dass die ermittelten Referenzpunkt-Raumkoordinaten zu einer Form verbunden werden, welche insbesondere eine Kugelform besitzt. Aus dem erstellten Modell des Oberschenkelkopfes kann eine Grund- oder Ausgangsgröße eines Modells des Oberschenkelimplantats ermittelt werden. Beispielsweise kann aus der Größe des Oberschenkelkopfes oder aus dem Durchmesser des Modells des Oberschenkelkopfes auf die Größe des Modells des Oberschenkelimplantats geschlossen werden. Insbesondere kann der Durchmesser des Modells des Oberschenkelkopfes als Grundgröße oder als Grundwert des Innendurchmessers des Modells des Oberschenkelimplantats verwendet werden. Basierend auf dem ermittelten Grundwert des Innendurchmessers des Modells des Oberschenkelimplantats oder der ermittelten Grundgröße kann ein Modell des Oberschenkelimplantats bestimmt werden. Dabei wird vorzugsweise das Ausgangsmodell des Oberschenkelimplantats nicht mit der Grundgröße oder dem ermittelten Grundwert erstellt, sondern es wird vorzugsweise eine Startgröße oder ein Startwert des Innendurchmessers des Modells des Oberschenkelimplantats verwendet, welcher um einen vorgegebenen Wert von dem Grundwert oder der Grundgröße abweicht und insbesondere um einen vorgegebenen Wert kleiner ist als der Grundwert oder die Grundgröße. Beispielsweise kann als Startgröße des Modells des Oberschenkelimplantats eine Größe gewählt werden, welche um eine Größeneinheit oder zwei oder mehr Größeneinheiten kleiner ist als die Grundgröße des Modells des Oberschenkelimplantats, welche aus der Größe des Oberschenkelkopfes ermittelt wurde. Auch kann als Startwert des Innendurchmessers des Modells des Oberschenkelimplantats ein Wert verwendet werden, welcher um eine Einheit oder zwei oder mehr Einheiten kleiner ist als der Grundwert des Innendurchmessers des Modells des Oberschenkelimplantats, welcher aus dem Innendurchmesser des Oberschenkelkopfes ermittelt wurde und vorzugsweise dem Innendurchmesser des Oberschenkelkopfes entspricht.

Das ermittelte virtuelle oder digitale Modell des Oberschenkelimplantats wird in dem virtuellen oder digitalen Modell des Oberschenkelkopfes platziert oder positioniert oder auf diesem virtuell aufzementiert. In der aufzementierten Position wird mittels des Navigationssystems ermittelt, ob mit dem Startwert eine passende Größe des Implantats gewählt wurde. Hierfür kann zum Beispiel ein absoluter Implantatwert ermittelt werden, welcher angibt, wie viele der ermittelten Referenzpunkt-Raumpositionen oder der Punkte des Modells des Oberschenkelkopfes innerhalb oder außerhalb des Modells des Oberschenkelimplantats liegen. Auch kann als absoluter Implantatwert ein Wert ermittelt werden, welcher angibt, wie viele der ermittelten Referenzpunkt-Raumpositionen oder der Punkte des Modells des Oberschenkelkopfes innerhalb oder außerhalb des Innendurchmessers des Modells des Oberschenkelimplantats liegen. Auch kann ein relativer Implantatwert ermittelt werden, welcher angibt, welcher Anteil der ermittelten Referenzpunkt-Raumpositionen oder der Punkte des Oberschenkelkopfes innerhalb oder außerhalb des Modells des Oberschenkelimplantats liegt oder welcher angibt, welcher Anteil der ermittelten Referenzpunkt-Raumpositionen oder Punkte des Modells des Oberschenkelkopfes innerhalb oder außerhalb des Innendurchmessers des Modells des Oberschenkelimplantats liegt. Vorzugsweise wird ein absoluter Implantatwert ermittelt, welcher angibt, wie viele der Referenzpunkt-Raumpositionen außerhalb des Innendurchmessers des Modells des Oberschenkelimplantats liegen oder ein relativer Implantatwert, welcher angibt, welcher Anteil der ermittelten Referenzpunkt-Raumpositionen außerhalb des Innendurchmessers des Modells des Oberschenkelimplantats liegt.

Der ermittelte Implantatwert wird mit einem vorgegebenen oder einem, zum Beispiel aus dem Grundwert des Oberschenkelimplantats abgeleiteten, Grenzwert verglichen. Wird bei einem Implantatwert, welcher angibt, wie viele oder welcher Anteil der ermittelten Referenzpunkt-Raumpositionen außerhalb des Innendurchmessers des Modells des Oberschenkelimplantats liegen bzw. liegt, der Grenzwert überschritten, so kann daraus geschlossen werden, dass das gewählte Modell des Oberschenkelimplantats zu klein für den vorhandenen Oberschenkelkopf ist oder an einer falschen Position angeordnet ist. In diesem Fall, kann das Modell des Oberschenkelimplantats virtuell, beispielsweise durch Verschieben, in eine weitere vorgegebene oder ermittelbare Position gebracht werden. In dieser neuen Position des Modells des Oberschenkelimplantats wird wiederum ein Implantatwert ermittelt, welcher mit dem vorgegebenen Grenzwert verglichen wird, wobei wieder aus dem Vergleich mit dem Grenzwert geschlossen werden kann, ob eine passende Größe und Position des Oberschenkelimplantats gefunden wurde oder ob das Oberschenkelimplantat wieder neu positioniert werden soll und wiederum auf passende Größe und Form überprüft werden soll. Bei einer Unterschreitung des Grenzwertes wird darauf geschlossen, dass das Modell des Oberschenkelimplantats eine richtige Größe aufweist und richtig positioniert ist, so dass unter Berücksichtigung dieser Daten ein Operateur oder Chirurg ein Oberschenkelimplantat beispielsweise mit der ermittelten Größe an der ermittelten Position positionieren kann.

Wird in keiner der Positionen der Grenzwert unterschritten oder wird in jeder Position der Grenzwert überschritten, so wird bevorzugt ein neues Modell des Oberschenkelimplantats mit einer größeren Größe oder einem größeren Innendurchmesser als der vorhergehenden Größe bzw. dem vorhergehenden Innendurchmesser berechnet. Beispielsweise kann der Innendurchmesser des Modells des Oberschenkelimplantats um eine oder zwei oder mehr Einheiten vergrößert werden. Das größere Modell des Oberschenkelimplantats wird vorzugsweise wiederum positioniert und auf seine Größe und Position mittels des Implantatwerts an verschiedenen Positionen überprüft.

Beispielsweise kann die Ermittlung eines passenden Modells des Oberschenkelimplantats solange durchgeführt werden, bis ein passendes oder das erste passende Oberschenkelimplantat gefunden wird oder es können alle passenden Größen und Positionen des Oberschenkelimplantats ermittelt werden, welche die vorgegebenen Grenzwerte einhalten, wobei aus allen passenden Modellen ein ideales Oberschenkelimplantat von dem Navigationssystem ausgewählt und vorgeschlagen werden kann und/oder von einem Operateur gewählt werden kann.

Vorzugsweise wird auch aus den ermittelten Referenzpunkt-Raumpositionen, insbesondere aus denen auf dem Oberschenkelhals ermittelten Referenzpunkt-Raumpositionen, ein Modell der Oberschenkelhalsachse des Oberschenkelhalses bestimmt. Das Modell der Oberschenkelhalsachse wird dabei vorzugsweise als Schnittgerade mindestens zweier Ebenen berechnet. Beispielsweise können die Ebenen anterior, posterior, inferior oder superior durch den Körper des Patienten verlaufende Ebenen sein.

Auch kann aus den Referenzpunkt-Raumpositionen, insbesondere den Referenzpunkt-Raumpositionen an der Schnittstelle zwischen Oberschenkelhals und Oberschenkelknochen, in den Modellen des Oberschenkelhalses und des Oberschenkelknochens ein Modell einer Ebene durch die Verbindung zwischen Oberschenkelhals und Oberschenkelknochen ermittelt werden, welche zum Beispiel mit der Oberschenkelhalsachse ein dreidimensionales Koordinatensystem aufspannen oder bilden kann, welches als Koordinatensystem verwendet werden kann, bezüglich dessen Berechnungen, wie die Implantatwertberechnungen, des Navigationssystems ausgeführt werden können.

Zur Neupositionierung des Modells des Oberschenkelimplantats zur Ermittlung einer geeigneten Position oder einer passenden Position des Oberschenkelimplantats kann das Modell des Oberschenkelimplantats verschoben werden. Vorzugsweise wird das Modell des Oberschenkelimplantats so verschoben, dass die Mittelachse oder die Oberschenkelhalsachse durch das Implantat und den Oberschenkelkopf nicht translatorisch verschoben wird oder keine translatorische Bewegung ausführt. Bevorzugt wird das Modell des Oberschenkelimplantats in eine Richtung, insbesondere anterior, superior, posterior oder inferior, verschoben, welche senkrecht zu dem Modell der Oberschenkelhalsachse ist, so dass es senkrecht zu der Mittelachse bzw. Oberschenkelhalsachse translatorisch bewegt wird, nicht jedoch entlang der Mittelachse bzw. Oberschenkelhalsachse. Somit kann das Modell des Oberschenkelimplantats umpositioniert oder neu positioniert werden, indem die Lage des Modells der Oberschenkelhalsachse in dem Modell des Oberschenkelhalses und/oder des Oberschenkelkopfes geändert wird.

Die Erfindung bezieht sich des Weiteren auf ein Computerprogramm, welches, wenn es in einen Computer geladen wird oder auf einem Computer läuft, ein wie oben beschriebenes Verfahren durchführt. Weiterhin bezieht sich die Erfindung auf ein Programmspeichermedium oder ein Computerprogrammprodukt mit einem solchen Programm.

Die erfindungsgemäße Vorrichtung umfasst ein Navigationssystem mit mindestens einer Kamera, insbesondere einer IR-Kamera, eine Erfassungseinheit, welche mit dem Navigationssystem drahtlos oder drahtgebunden verbunden oder in das Navigationssystem integriert sein kann, sowie eine Recheneinheit, welche mit dem Navigationssystem drahtlos oder drahtgebunden verbunden oder in das Navigationssystem integriert sein kann. Mit der Erfassungseinheit, wie einem Pointer oder Laser-Pointer oder einer Kamera, werden Referenzpunkte auf einem Oberschenkelhals und/oder einem Oberschenkelkopf eines Patienten erfasst oder detektiert, wobei insbesondere anatomische Landmarken des Oberschenkelhalses und/oder des Oberschenkelkopfes erfasst werden. Mit der Kamera des Navigationssystems kann die Position eines an dem Oberschenkelhals angeordneten Referenzsterns, insbesondere eines Infrarot-Strahlung emittierenden oder reflektierenden Referenzsterns, im Raum erfasst oder verfolgt werden.

Mit der Recheneinheit werden aus den erfassten Referenzpunkten unter Berücksichtigung der ermittelten Positionen des Referenzsterns dreidimensionale Referenzpunkt-Raumpositionen der erfassten Referenzpunkte ermittelt mittels derer der Oberschenkelhals und/oder der Oberschenkelkopf registriert werden können. Aus den registrierten Daten oder den dreidimensionalen Referenzpunkt-Raumpositionen erstellt die Recheneinheit ein Modell des Oberschenkelhalses und/oder des Oberschenkelkopfes, wie einen kugelförmigen Oberschenkelkopf, oder erstellt ein angenähertes virtuelles oder digitales Modell des Oberschenkelhalses und/oder des Oberschenkelkopfes. Basierend auf dem erstellten Modell des Oberschenkelkopfes bestimmt die Recheneinheit eine Grundgröße eines Modells des Oberschenkelimplantats. Zum Beispiel kann die Recheneinheit aus den Referenzpunkt-Raumpositionen den Durchmesser des Oberschenkelkopfes berechnen und diesen für die Ermittlung der Grundgröße des Modells des Oberschenkelimplantates verwenden. Vorzugsweise wird als Grundwert des Innendurchmessers des Modells des Oberschenkelimplantats der ermittelte Durchmesser des Oberschenkelkopfes verwendet. Basierend auf der ermittelten Grundgröße des Oberschenkelimplantats erstellt die Recheneinheit ein Modell des Oberschenkelimplantats. Bevorzugt kann als Startwert oder Startgröße des Modells des Oberschenkelimplantats eine von der Grundgröße abweichende Startgröße verwendet werden, welche vorzugsweise um eine oder zwei oder mehr Einheiten kleiner ist als die Grundgröße des Modells des Oberschenkelimplantats. Insbesondere kann als Startwert des Innendurchmessers des Modells des Oberschenkelimplantats ein von dem ermittelten Grundwert des Innendurchmessers abweichender Innendurchmesser verwendet werden, wobei als Startwert des Innendurchmessers des Modells des Oberschenkelimplantats vorzugsweise ein um ein, zwei oder mehr Einheiten kleinerer Innendurchmesser von der Recheneinheit verwendet wird.

Das mit den Startwerten oder der Startgröße erstellte Modell des Oberschenkelimplantats wird virtuell von der Recheneinheit in oder an dem Modell des Oberschenkelkopfes platziert oder positioniert, wobei diese virtuelle Positionierung auch grafisch auf einer Anzeigevorrichtung dargestellt werden kann. Weiter werden mit der Recheneinheit die ermittelten Referenzpunkt-Raumpositionen oder Punkte des Modells des Oberschenkelkopfes mit dem Modell des Oberschenkelimplantats verglichen, wobei insbesondere ermittelt wird, ob das Modell des Oberschenkelimplantats ausreichend groß für das Modell des Oberschenkelkopfes ist. Dabei wird bevorzugt von der Recheneinheit ein Implantatwert ermittelt, welcher angibt, wie viele oder welcher Anteil der ermittelten Referenzpunkt-Raumpositionen oder Punkte des Modells des Oberschenkelkopfes außerhalb des Modells des Oberschenkelimplantats liegen bzw. liegt oder außerhalb des Innendurchmessers des Oberschenkelimplantats liegen bzw. liegt. Liegt der ermittelte Implantatwert über einem vorgegebenen Grenzwert, so wird das Modell des Oberschenkelimplantats umpositioniert und es wird ein neuer Implantatwert ermittelt oder es wird ein größeres Modell des Oberschenkelimplantats ermittelt und dessen Implantatwert wird vorzugsweise an verschiedenen Positionen bestimmt. Die Recheneinheit stellt eine passende oder mögliche Größe und Position des Oberschenkelimplantats vorzugsweise dann fest, wenn der Implantatwert den Grenzwert nicht überschreitet. Das passende Oberschenkelimplantat wird oder die passenden Oberschenkelimplantate werden von der Recheneinheit vorzugsweise auf einer Anzeigevorrichtung ausgegeben oder dargestellt, so dass ein Operateur das ideale Oberschenkelimplantat auswählen kann.

Die Erfindung wird nachfolgend anhand bevorzugter Ausführungsformen beschrieben werden. Es zeigen:
- Figur 1: ein Ablaufdiagramm einer Ausführungsform des erfindungsgemäße Verfahrens;
- Figur 2: die Erfassung von Referenzpunkten eines Oberschenkelkopfes;
- Figur 3: ein virtuelles Modell des Oberschenkelhalses, des Oberschenkelkopfes und des Oberschenkelimplantats;
- Figur 3A: das virtuelle Koodinatensystem der Anordnung aus Figur 3A; und
- Figur 4: ein Ablaufdiagram für Neupositionierungen gemäß einer Ausführungsform des erfindungsgemäßen Verfahrens.

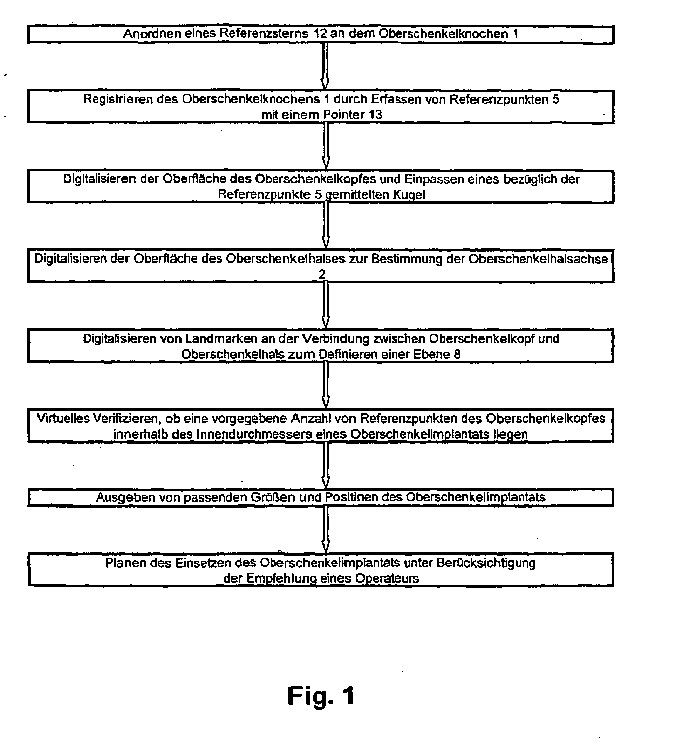

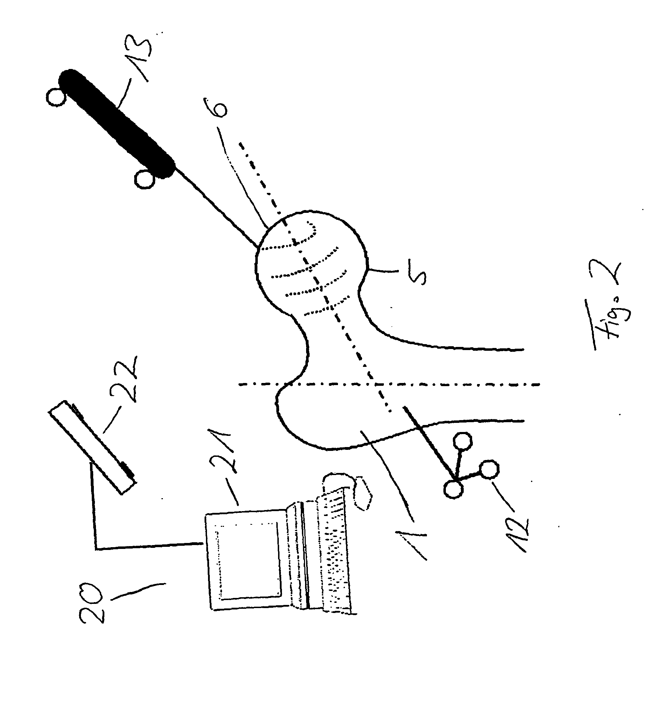

Figur 1 zeigt ein Ablaufdiagramm einer Ausführungsform des Verfahrens der vorliegenden Erfindung. Wie in Figur 2 gezeigt, wird an dem Oberschenkelhalsknochen 1 ein IR-Strahlung reflektierender oder emittierender Referenzstern 12 angeordnet, dessen Position von einer IR-Kamera 22 eines Navigationssystems 20 ermittelt werden kann. Mittels eines Pointers 13 wird die Oberfläche des abgefrästen Oberschenkelkopfes 6 abgetastet und Referenzpunkte 5 des Oberschenkelkopfes 6 werden erfasst. Die Position der Referenzpunkte im Raum wird von dem Navigationssystem 20 ermittelt. Somit kann der Oberschenkelknochen 1 registriert werden. Weiter wird mittels der erfassten Referenzpunke 5 die Oberfläche des Oberschenkelkopfes 6 digitalisiert, indem ein virtuelles Modell des Oberschenkelkopfes 6 gebildet wird. Dabei wird eine Form oder Oberfläche den ermittelten Referenzpunkten 5 angenähert und diese Form, wie eine Kugelform, wird so zwischen die ermittelten Referenzpunkte 5 eingepasst, dass der mittlere Fehler oder der mittlere Abstand der Referenzpunkte 5 von der Oberfläche der Kugel minimal wird. In einem nächsten Schritt wird die Oberfläche des Oberschenkelhalses 1 anhand der erfassten und ermittelten Referenzpunkte 5 auf dem Oberschenkelhals 1 digitalisiert. Aus dem digitalisierten Modell des Oberschenkelhalses 1 wird die Oberschenkelhalsachse 2 des Modells ermittelt.

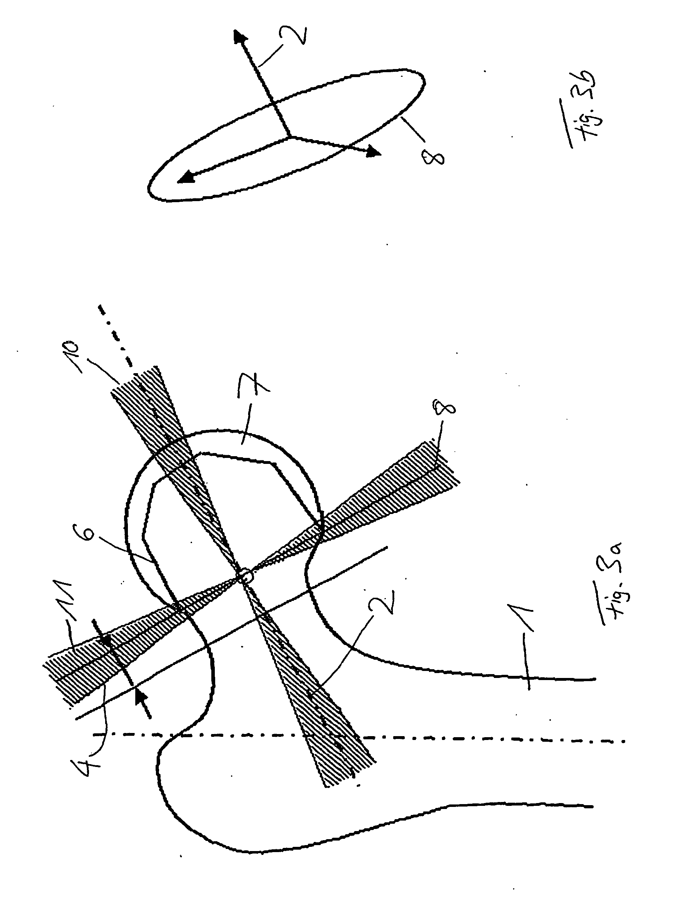

Aus den erfassten Punkten oder Landmarken an der Verbindung zwischen Oberschenkelkopf 6 und Oberschenkelhals 1 wird eine Verbindungsebene 8 ermittelt, welche durch die Verbindung zwischen Oberschenkelkopf 6 und Oberschenkelhals 1 verläuft. Durch die Ebene 8 werden zwei Raumrichtungen definiert, so dass aus der Ebene 8 und der Oberschenkelhalsachse 2 ein dreidimensionales Koordinatensystem, wie in Figur 3B zu sehen, ermittelt wird. Bezüglich des durch die Verbindungsebene 8 und die Oberschenkelhalsachse 2 definierten Koordinatensystems werden Berechnungen des Navigationssystems 20 durchgeführt. Anhand der vorliegenden Daten, wie den Referenzpunkten 5 des Oberschenkelkopfes 6 und dem Modell des Oberschenkelimplantats 7, kann das Navigationssystem 20 berechnen, ob eine vorgegebene Anzahl von Referenzpunkten 5 des Oberschenkelkopfes 6 innerhalb des Innendurchmessers des Oberschenkelimplantats 7 liegt. Liegen mehr Referenzpunkte 5 des Oberschenkelkopfes 6 innerhalb des Oberschenkelimplantats 7 als ein vorgegebener Schwellwert, so hat das Oberschenkelimplantat 7 eine ausreichende Größe, so dass die ausgewählte Größe und die ausgewählte Position des Oberschenkelimplantats 6 als passend eingestuft werden und die Daten des Oberschenkelimplantats 7 ausgegeben werden. Liegen weniger Referenzpunkte 5 des Oberschenkelkopfes 6 innerhalb des Innendurchmessers des Oberschenkelimplantats 7 als der vorgegebene Schwellwert, so wird seitens des Navigationssystems 20 angenommen, dass das Oberschenkelimplantat 7 zu klein gewählt und/oder falsch positioniert ist. Das Oberschenkelimplantat 7 wird anterior, superior, posterior oder inferior verschoben, so dass sich die Mittelachse oder Oberschenkelhalsachse 2 nicht translatorisch verschiebt, sondern, wie in Figur 3A gezeigt, ihre räumliche Lage ändert oder gedreht wird.. Dabei wird auch die Ebene 8 gedreht. In dieser neuen Position des Oberschenkelimplantats 7 wird wiederum ermittelt, ob der vorgegebene Schwellwert überschritten oder unterschritten wird. Liegen mehr Referenzpunkte 6 des Oberschenkelkopfes 7 außerhalb des Innendurchmessers des Oberschenkelimplantats 7, wird diese Position des Oberschenkelimplantats 7 als eine nicht mögliche oder passende Position zur Positionierung des untersuchten Oberflächenimplantats 7 angenommen. Wurden alle möglichen Positionen des Oberschenkelimplantats 7 untersucht, so wird virtuell die Größe oder der Innendurchmesser des Oberschenkelimplantats 7 erhöht und es wird erneut in den möglichen Positionen überprüft, ob der Schwellwert des größeren Modells des Oberflächenimplantats überschritten oder unterschritten wird.

Alle Größen und Formen, bei denen ermittelt wird, dass ausreichend viele Referenzpunkte 5 des Oberschenkelkopfes 6 innerhalb des Innendurchmessers des Oberschenkelimplantats 7 liegen, werden als passende Größen und Formen und Positionen des Oberschenkelimplantats 7 angenommen und ausgegeben. Aus diesen ausgegebenen passenden Größen und Positionen des Oberschenkelimplantats 7 kann der Operateur das ideale Oberschenkelimplantat 7 auswählen und einsetzen.

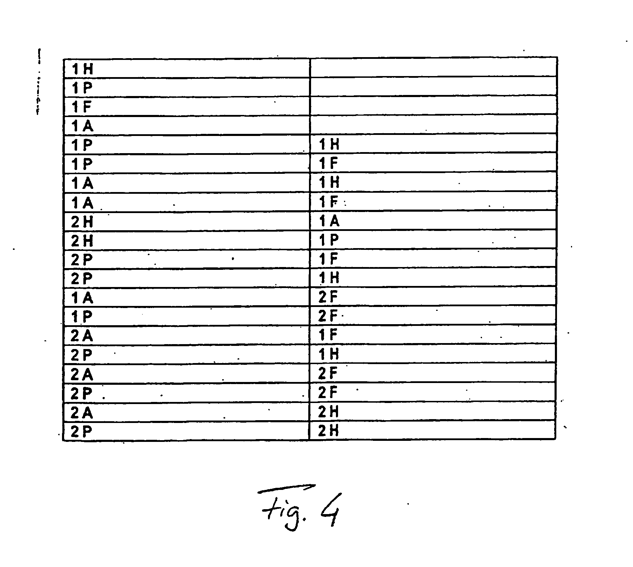

Figur 4 zeigt den Ablauf möglicher Verschiebungen oder Neupositionierungen eines Oberschenkelimplantats 7 einer bestimmten Größe. Das Modell des Oberflächenimplantats 7 einer Größe kann in alle in Figur 4 gezeigten Positionen verschoben werden und dort auf die Schwellwertüberschreitung getestet werden. Dabei bezeichnet der Buchstabe "H" eine Verschiebung in Kopfrichtung, der Buchstabe "F" eine Verschiebung in Fußrichtung, der Buchstabe "A" eine Verschiebung anterior und der Buchstabe "P" eine Verschiebung posterior, jeweils ausgehend von der vorherigen Position des Modells des Oberschenkelimplantats 7. Die Zahlenwerte geben an, wie stark oder um wie viele Einheiten das Oberschenkelimplantat 7 in die jeweils gewählte Richtung verschoben wird.

## Patentansprüche

1. Verfahren zur Auswahl eines Oberschenkelimplantats (7) basierend auf Modellen eines Oberschenkelhalses (1) und/oder eines Oberschenkelkopfes (6) eines Patienten mit den folgenden Schritten:
a) Erfassen von Referenzpunkten (5), insbesondere von anatomischen Landmarken, an dem Oberschenkelhals (1) und/oder dem Oberschenkelkopf (6) des Patienten;
b) Registrieren des Oberschenkelhalses (1) und/oder des Oberschenkelkopfes (6) basierend auf dreidimensionalen Referenzpunkt-Raumpositionen der erfassten Referenzpunkte (5);
c) Erstellen eines Modells des Oberschenkelhalses (1) und/oder des Oberschenkelkopfes (6) aus den dreidimensionalen Referenzpunkt-Raumpositionen;
d) Ermitteln einer Grundgröße eines Modells des Oberschenkelimplantats (7) basierend auf dem erstellten Modell des Oberschenkelkopfes (6);
e) Erstellen eines Modells des Oberschenkelimplantats (7) basierend auf der Grundgröße des Modells des Oberschenkelimplantats (7);
f) Positionierung des Modells des Oberschenkelimplantats (7) an eine Position in oder an dem Modell des Oberschenkelkopfes (6);
g) Ermitteln eines Implantatwerts, welcher angibt, wie viele oder welcher Anteil der ermittelten Referenzpunkt-Raumpositionen innerhalb des Modells des Oberschenkelimplantats (7) liegen beziehungsweise liegt;
h) Überprüfen, ob der Implantatwert einen vorgegebenen Grenzwert überschreitet; und
i) Neupositionieren des Modells des Oberschenkelimplantats (7) und Ausführen der Schritte g) bis i), wenn der Implantatwert den Grenzwert überschreitet, oder Feststellen einer passenden Größe und Position des Oberschenkelimplantats (7), wenn der Implantatwert den Grenzwert nicht überschreitet.

2. Verfahren nach dem vorhergehenden Anspruch, wobei die Schritte f) bis i) mit mindestens einem weiteren von dem auf der Grundgröße basierenden Modell verschiedenen Modell des Oberschenkelimplantats (7), insbesondere einem größeren Modell des Oberschenkelimplantats (7), wiederholt werden.

3. Verfahren nach einem dem vorhergehenden Anspruch, wobei das mindestens eine weitere Modell des Oberschenkelimplantats (7) einen größeren Innendurchmesser aufweist als das auf der Grundgröße basierende Modell.

4. Verfahren nach einem der vorhergehenden Ansprüche, wobei als die Grundgröße des Modells des Oberschenkelimplantats (7) der Grundwert des Innendurchmessers des Modells des Oberschenkelimplantats (7) verwendet wird, wobei als Grundwert des Innendurchmessers des Oberschenkelimplantats (7) der Durchmesser des erstellten Modells des Oberschenkelkopfes (6) verwendet wird.

5. Verfahren nach dem vorhergehenden Anspruch, wobei das Modell des Oberschenkelimplantats mit einem Startwert des Innendurchmessers des Modells des Oberschenkelimplantats (7) erstellt wird, wobei der Startwert des Innendurchmessers des Modells des Oberschenkelimplantats (7) um einen vorgegebenen Wert von dem Grundwert abweicht, insbesondere um einen vorgegebenen Wert kleiner ist als der Grundwert;

6. Verfahren nach einem der vorhergehenden Ansprüche, wobei als Implantatwert ermittelt wird, wie viele oder welcher Anteil der ermittelten Referenzpunkt-Raumpositionen außerhalb des Innendurchmessers des Modells des Oberschenkelimplantats (7) liegen;

7. Verfahren nach einem der vorhergehenden Ansprüche, wobei das Modell des Oberschenkelkopfes (6) durch Ermitteln einer Form, insbesondere einer Kugelform, oder Oberfläche aus den Referenzpunkt-Raumpositionen gebildet wird.

8. Verfahren nach einem der vorhergehenden Ansprüche, wobei aus den Referenzpunkt-Raumpositionen ein Modell der Oberschenkelhalsachse (2) ermittelt wird.

9. Verfahren nach dem vorhergehenden Anspruch, wobei das Modell der Oberschenkelhalsachse (2) als Schnittgerade zumindest zweier Ebenen ermittelt wird, wobei die Ebenen insbesondere anterior, superior, posterior und inferior durch den Patienten verlaufen.

10. Verfahren nach einem der vorhergehenden Ansprüche, wobei aus den Referenzpunkt-Raumpositionen, insbesondere aus Referenzpunkt-Raumpositionen der Verbindung zwischen Oberschenkelhals (1) und Oberschenkelkopf, in den Modellen des Oberschenkelhalses (1) und des Oberschenkelkopfes (6) eine Ebene durch die Verbindung zwischen Oberschenkelhals (1) und Oberschenkelkopf (6) ermittelt wird.

11. Verfahren nach einem der vorhergehenden Ansprüche, wobei zur Neupositionierung des Modells des Oberschenkelimplantats (7) das Modell des Oberschenkelimplantats (7) in eine Richtung, insbesondere anterior, superior, posterior oder inferior, verschoben wird, welche senkrecht zu dem Modell der Oberschenkelhalsachse (2) ist.

12. Verfahren nach einem der vorhergehenden Ansprüche, wobei das Modell des Oberschenkelimplantats (7) zur Neupositionierung relativ zu dem Modell des Oberschenkelkopfes (6) in allen Freiheitsgraden verschiebbar ist.

13. Verfahren nach einem der vorhergehenden Ansprüche, wobei zur Neupositionierung des Oberschenkelimplantats (7) die Lage des Modells der Oberschenkelhalsachse (2) in dem Modell des Oberschenkelhalses (1) relativ zu dem Modell des Oberschenkelimplantats (7) geändert wird.

14. Verfahren nach einem der vorhergehenden Ansprüche, wobei eine Vielzahl passender Formen und Größen des Oberschenkelimplantats (7) ermittelt und ausgegeben wird.

15. Verfahren nach einem der vorhergehenden Ansprüche, wobei eine ideal passende Größe und Form des Oberschenkelimplantates (7) ermittelt und ausgegeben wird.

16. Computerprogramm, welches, wenn es auf einem Computer läuft oder in einen Computer geladen ist, das Verfahren nach einem der vorhergehenden Ansprüche durchführt.

17. Programmspeichermedium oder Computerprogrammprodukt mit dem Computerprogramm nach dem vorhergehenden Anspruch.

18. Vorrichtung zur Auswahl eines Oberschenkelimplantats (7) basierend auf Modellen eines Oberschenkelhalses (1) und/oder eines Oberschenkelkopfes (6) eines Patienten mit einer Erfassungseinheit (13) zum Erfassen von Referenzpunkten (5), insbesondere von anatomischen Landmarken, an dem Oberschenkelhals (1) und/oder dem Oberschenkelkopf (6) des Patienten, einem mit der Erfassungseinheit (13) verbundenen Navigationssystem (20) mit mindestens einer Kamera (22), insbesondere einer IR-Kamera, zum Erfassen der Position eines an dem Oberschenkelhals (1) angeordneten Referenzsterns (12) und einer mit dem Navigationssystem (20) verbundenen Recheneinheit (21) zum:
a) Registrieren des Oberschenkelhalses (1) und/oder des Oberschenkelkopfes (6) basierend auf dreidimensionalen Referenzpunkt-Raumpositionen der erfassten Referenzpunkte (5);
b) Erstellen eines Modells des Oberschenkelhalses (1) und/oder des Oberschenkelkopfes (6) aus den dreidimensionalen Referenzpunkt-Raumpositionen;
c) Ermitteln einer Grundgröße eines Modells des Oberschenkelimplantats (7) basierend auf dem erstellten Modell des Oberschenkelkopfes (6);
d) Erstellen eines Modells des Oberschenkelimplantats (7) basierend auf der Grundgröße des Modells des Oberschenkelimplantats (7);
e) Ermitteln eines Implantatwerts, welcher angibt, wie viele oder welcher Anteil der ermittelten Referenzpunkt-Raumpositionen außerhalb des Innendurchmessers des Modells des Oberschenkelimplantats (7) liegen beziehungsweise liegt;
f) Überprüfen, ob der Implantatwert einen vorgegebenen Grenzwert überschreitet; und
g) Feststellen einer passenden Größe und Position des Oberschenkelimplantats (7), wenn der Implantatwert den Grenzwert nicht überschreitet.

19. Vorrichtung nach dem vorhergehenden Anspruch mit einer mit dem Navigationssystem (20) verbundenen Ausgabevorrichtung zur grafischen Darstellung der Modelle des Oberschenkelhalses (1), des Oberschenkelkopfes (6) und des Oberschenkelimplantats (7).

## Claims

1. A method for selecting a femoral implant (7) on the basis of models of a femoral neck (1) and/or a femoral head (6) of a patient, comprising the following steps:
a) detecting reference points (5), in particular anatomical landmarks, on the femoral neck (1) and/or femoral head (6) of the patient;
b) registering the femoral neck (1) and/or femoral head (6) on the basis of three-dimensional reference point spatial positions of the detected reference points (5);
c) producing a model of the femoral neck (1) and/or femoral head (6) from the three-dimensional reference point spatial positions;
d) ascertaining a base size of a model of the femoral implant (7) on the basis of the produced model of the femoral head (6);
e) producing a model of the femoral implant (7) on the basis of the base size of the model of the femoral implant (7);
f) positioning the model of the femoral implant (7) at a position in or on the model of the femoral head (6);
g) ascertaining an implant value which indicates how many or what proportion of the ascertained reference point spatial positions are inside the model of the femoral implant (7);
h) checking whether the implant value exceeds a predetermined limit value; and
i) repositioning the model of the femoral implant (7) and performing Steps g) to i) if the implant value exceeds the limit value, or determining an appropriate size and position of the femoral implant (7) if the implant value does not exceed the limit value.

2. The method according to the preceding claim, wherein Steps f) to i) are repeated using at least one other model of the femoral implant (7), different from the model based on the base size, in particular a larger model of the femoral implant (7).

3. The method according to any one of the preceding claims, wherein the at least one other model of the femoral implant (7) exhibits a larger inner diameter than the model based on the base size.

4. The method according to any one of the preceding claims, wherein the base value of the inner diameter of the model of the femoral implant (7) is used as the base size of the model of the femoral implant (7), wherein the diameter of the produced model of the femoral head (6) is used as the base value of the inner diameter of the femoral implant (7).

5. The method according to the preceding claim, wherein the model of the femoral implant is produced using a starting value of the inner diameter of the model of the femoral implant (7), wherein the starting value of the inner diameter of the model of the femoral implant (7) deviates from the base value by a predetermined value and is in particular smaller than the base value by a predetermined value.

6. The method according to any one of the preceding claims, wherein the implant value is ascertained as how many or what proportion of the ascertained reference point spatial positions are outside the inner diameter of the model of the femoral implant (7).

7. The method according to any one of the preceding claims, wherein the model of the femoral head (6) is formed by ascertaining a shape, in particular a spherical shape, or a surface from the reference point spatial positions.

8. The method according to any one of the preceding claims, wherein a model of the femoral neck axis (2) is ascertained from the reference point spatial positions.

9. The method according to the preceding claim, wherein the model of the femoral neck axis (2) is ascertained as an intersecting straight line of at least two planes, wherein the planes run in particular anteriorly, superiorly, posteriorly and inferiorly through the patient.

10. The method according to any one of the preceding claims, wherein from the reference point spatial positions, in particular reference point spatial positions of the connection between the femoral neck (1) and the femoral head, a plane through the connection between the femoral neck (1) and the femoral head (6) is ascertained in the models of the femoral neck (1) and the femoral head (6).

11. The method according to any one of the preceding claims, wherein in order to reposition the model of the femoral implant (7), the model of the femoral implant (7) is shifted, in particular anteriorly, superiorly, posteriorly or inferiorly, in a direction which is perpendicular to the model of the femoral neck axis (2).

12. The method according to any one of the preceding claims, wherein for repositioning, the model of the femoral implant (7) can be shifted in all degrees of freedom relative to the model of the femoral head (6).

13. The method according to any one of the preceding claims, wherein in order to reposition the femoral implant (7), the position of the model of the femoral neck axis (2) is changed relative to the model of the femoral implant (7), in the model of the femoral neck (1).

14. The method according to any one of the preceding claims, wherein a number of appropriate shapes and sizes of the femoral implant (7) are ascertained and outputted.

15. The method according to any one of the preceding claims, wherein an ideally appropriate size and shape of the femoral implant (7) is ascertained and outputted.

16. A computer program which, when it is running on a computer or is loaded onto a computer, performs the method according to any one of the preceding claims.

17. A program storage medium or computer program product comprising the computer program according to the preceding claim.

18. A device for selecting a femoral implant (7) on the basis of models of a femoral neck (1) and/or a femoral head (6) of a patient, comprising: a detection unit (13) for detecting ' reference points (5), in particular anatomical landmarks, on the femoral neck (1) and/or femoral head (6) of the patient; a navigation system (20) connected to the detection unit (13) and comprising at least one camera (22), in particular an infrared camera, for detecting the position of a reference star (12) arranged on the femoral neck (1); and a computational unit (21) connected to the navigation system (20), for:
a) registering the femoral neck (1) and/or femoral head (6) on the basis of three-dimensional reference point spatial positions of the detected reference points (5);
b) producing a model of the femoral neck (1) and/or femoral head (6) from the three-dimensional reference point spatial positions;
c) ascertaining a base size of a model of the femoral implant (7) on the basis of the produced model of the femoral head (6);
d) producing a model of the femoral implant (7) on the basis of the base size of the model of the femoral implant (7);
e) ascertaining an implant value which indicates how many or what proportion of the ascertained reference point spatial positions are outside the inner diameter of the model of the femoral implant (7);
f) checking whether the implant value exceeds a predetermined limit value; and
g) determining an appropriate size and position of the femoral implant (7) if the implant value does not exceed the limit value.

19. The device according to the preceding claim, comprising an output device, connected to the navigation system (20), for graphically displaying the models of the femoral neck (1), the femoral head (6) and the femoral implant (7).

## Revendications

1. Procédé pour sélectionner un implant fémoral (7) sur la base de modèles d'un col fémoral (1) et/ou d'une tête fémorale (6) d'un patient, comportant les étapes suivantes consistant à :
a) détecter des points de référence (5), en particulier de points de repère, sur le col fémoral (1) et/ou la tête fémorale (6) du patient,
b) repérer le col fémoral (1) et/ou la tête fémorale (6) sur la base de positions spatiales tridimensionnelles des points de référence des points de référence (5) détectés,
c) établir un modèle du col fémoral (1) et/ou de la tête fémorale (6) à partir des positions spatiales tridimensionnelles des points de référence,
d) déterminer une taille de base d'un modèle de l'implant fémoral (7) sur la base du modèle établi de la tête fémorale (6),
e) établir un modèle de l'implant fémoral (7) sur la base de la taille de base du modèle de l'implant fémoral (7),
f) positionner le modèle de l'implant fémoral (7) en une position dans ou sur le modèle de la tête fémorale (6),
g) déterminer une valeur d'implant qui indique le nombre ou la proportion des positions spatiales établies des points de référence qui est ou qui sont à l'intérieur du modèle de l'implant fémoral (7),
h) vérifier si la valeur d'implant dépasse une valeur limite prédéterminée, et
i) repositionner le modèle de l'implant fémoral (7) et exécuter les étapes g) à i) si la valeur d'implant dépasse la valeur limite, ou définir une taille et une position appropriées de l'implant fémoral (7) si la valeur d'implant ne dépasse pas la valeur limite.

2. Procédé selon la revendication précédente, dans lequel les étapes f) à i) sont répétées avec au moins un modèle supplémentaire de l'implant fémoral (7) différent du modèle basé sur la taille de base, en particulier un modèle plus grand de l'implant fémoral (7).

3. Procédé selon l'une quelconque des revendications précédentes, dans lequel le au moins un modèle supplémentaire de l'implant fémoral (7) présente un diamètre intérieur plus grand que le modèle basé sur la taille de base.

4. Procédé selon l'une quelconque des revendications précédentes, dans lequel la valeur de base du diamètre intérieur du modèle de l'implant fémoral (7) est utilisée comme taille de base du modèle de l'implant fémoral (7), le diamètre du modèle établi de la tête fémorale (6) étant utilisé comme valeur de base du diamètre intérieur de l'implant fémoral (7).

5. Procédé selon la revendication précédente, dans lequel le modèle de l'implant fémoral est établi à l'aide d'une valeur initiale du diamètre intérieur du modèle de l'implant fémoral (7), la valeur initiale du diamètre intérieur du modèle de l'implant fémoral (7) s'écartant de la valeur de base d'une valeur prédéfinie, en particulier d'une valeur prédéfinie inférieure à la valeur de base.

6. Procédé selon l'une quelconque des revendications précédentes, dans lequel le nombre ou la proportion des positions spatiales établies des points de référence qui sont à l'extérieur du diamètre intérieur du modèle de l'implant fémoral (7), est déterminé en tant que valeur d'implant.

7. Procédé selon l'une quelconque des revendications précédentes, dans lequel le modèle de la tête fémorale (6) est formé en déterminant une forme, en particulier une forme sphérique, ou une surface à partir des positions spatiales des points de référence.

8. Procédé selon l'une quelconque des revendications précédentes, dans lequel un modèle de l'axe de col fémoral (2) est déterminé à partir des positions spatiales des points de référence.

9. Procédé selon la revendication précédente, dans lequel le modèle de l'axe de col fémoral (2) est déterminé sous forme de droite d'intersection d'au moins deux plans, dans lequel les plans passent à travers le patient, en particulier antérieurement, supérieurement, postérieurement et inférieurement.

10. Procédé selon l'une quelconque des revendications précédentes, dans lequel un plan à travers la liaison entre le col fémoral (1) et la tête fémorale (6) est déterminé à partir des positions spatiales des points de référence, en particulier à partir des positions spatiales des points de référence de la liaison entre le col fémoral (1) et la tête fémorale (6), dans les modèles du col fémoral (1) et de la tête fémorale (6).

11. Procédé selon l'une quelconque des revendications précédentes, dans lequel, pour repositionner le modèle de l'implant fémoral (7), le modèle de l'implant fémoral (7) est déplacé dans une direction, en particulier antérieurement, supérieurement, postérieurement ou inférieurement, qui est perpendiculaire au modèle de l'axe de col fémoral (2).

12. Procédé selon l'une quelconque des revendications précédentes, dans lequel le modèle de l'implant fémoral (7) peut être déplacé dans tous les degrés de liberté afin de le repositionner par rapport au modèle de la tête fémorale (6).

13. Procédé selon l'une quelconque des revendications précédentes, dans lequel, pour repositionner l'implant fémoral (7), la position du modèle de l'axe de col fémoral (2) dans le modèle du col fémoral (1) est changée par rapport au modèle de l'implant fémoral (7).

14. Procédé selon l'une quelconque des revendications précédentes, dans lequel une pluralité de formes et de tailles appropriées de l'implant fémoral (7) est déterminée et générée en sortie.

15. Procédé selon l'une quelconque des revendications précédentes, dans lequel une taille et une forme idéalement appropriées de l'implant fémoral (7) sont déterminées et générées en sortie.

16. Programme informatique qui, lorsqu'il tourne sur un ordinateur ou lorsqu'il est chargé dans un ordinateur, met en oeuvre le procédé selon l'une quelconque des revendications précédentes.

17. Support de mémorisation de programme ou produit de programme informatique comportant le programme informatique selon la revendication précédente.

18. Dispositif pour sélectionner un implant fémoral (7) sur la base de modèles d'un col fémoral (1) et/ou d'une tête fémorale (6) d'un patient, comportant une unité de détection (13) pour détecter des points de référence (5), en particulier des points de repère, sur le col fémoral (1) et/ou la tête fémorale (6) du patient, un système de navigation (20) relié à l'unité de détection (13) comportant au moins une caméra (22), en particulier une caméra IR, pour détecter la position d'une étoile de référence (12) disposée sur le col fémoral (1), et une unité de calcul (21) reliée au système de navigation (20) pour :
a) repérer le col fémoral (1) et/ou la tête fémorale (6) sur la base de positions spatiales tridimensionnelles de points de référence des points de référence détectés (5),
b) établir un modèle du col fémoral (1) et/ou de la tête fémorale (6) à partir des positions spatiales tridimensionnelles des points de référence,
c) déterminer une taille de base d'un modèle de l'implant fémoral (7) sur la base du modèle établi de la tête fémorale (6),
d) établir un modèle de l'implant fémoral (7) sur la base de la taille de base du modèle de l'implant fémoral (7),
e) déterminer une valeur d'implant qui indique le nombre ou la proportion des positions spatiales de points de référence qui est ou qui sont à l'extérieur du diamètre intérieur du modèle de l'implant fémoral (7),
f) vérifier si la valeur d'implant dépasse une valeur limite prédéfinie, et
g) définir une taille et une position appropriées de l'implant fémoral (7) si la valeur d'implant ne dépasse pas la valeur limite.

19. Dispositif selon la revendication précédente comportant un dispositif de sortie relié au système de navigation (20) pour la représentation graphique des modèles du col fémoral (1), de la tête fémorale (6) et de l'implant fémoral (7).
